(19) **Europäisches Patentamt European Patent Office Office européen des brevets**

(11) **EP 2 029 505 B1**

(12) **FASCICULE DE BREVET EUROPEEN**

(45) Date de publication et mention
de la délivrance du brevet:
**16.03.2011 Bulletin 2011/11**

(51) Int Cl.:
*C07C 5/27* $^{(2006.01)}$     *B01J 29/74* $^{(2006.01)}$

(21) Numéro de dépôt: **07731437.5**

(22) Date de dépôt: **10.05.2007**

(86) Numéro de dépôt international:
**PCT/FR2007/000796**

(87) Numéro de publication internationale:
**WO 2007/138176 (06.12.2007 Gazette 2007/49)**

(54) **UTILISATION D'UN CATALYSEUR À BASE DE ZÉOLITHE ITQ-6 EN ISOMÉRISATION D'UNE COUPE C8 AROMATIQUE**

VERWENDUNG EINES ITQ-6-ZEOLITKATALYSATORS BEI DER ISOMERISIERUNG EINER AROMATISCHEN C8-FRAKTION

USE OF AN ITQ-6 ZEOLITE CATALYST IN AN AROMATIC C8 CUT ISOMERIZATION

(84) Etats contractants désignés:
**BE DE ES NL**

(30) Priorité: **30.05.2006 FR 0604927**

(43) Date de publication de la demande:
**04.03.2009 Bulletin 2009/10**

(73) Titulaire: **IFP Energies Nouvelles
92852 Rueil-Malmaison Cedex (FR)**

(72) Inventeurs:
• **MABILON, Gil
69390 Charly (FR)**
• **GUILLON, Emmanuelle
69390 Vernaison (FR)**
• **SANCHEZ, Eric
69230 Saint Genis Laval (FR)**

(56) Documents cités:
**US-A1- 2005 020 435     US-B1- 6 469 226**

• **A.CORMA ET AL.: "New Aluminosilicate and Titanosilicate Delaminated Materials Active for Acid Catalysis, and Oxidation Reactions Using H2O2" JOURNAL OF AMERICAN CHEMICAL SOCIETY., vol. 122, 2000, pages 2804-2809, XP002397887**
• **AYALA V ET AL: "Hybrid organic-inorganic catalysts: a cooperative effect between support, and palladium and nickel salen complexes on catalytic hydrogenation of imines" JOURNAL OF CATALYSIS, ACADEMIC PRESS, DULUTH, MN, US, vol. 224, no. 1, 15 mai 2004 (2004-05-15), pages 170-177, XP004504127 ISSN: 0021-9517**
• **CORMA A ET AL: "Influence of Pore-Volume Topology of Zeolite ITQ-7 in Alkylation and Isomerization of Aromatic Compounds" JOURNAL OF CATALYSIS, ACADEMIC PRESS, DULUTH, MN, US, vol. 207, no. 1, 1 avril 2002 (2002-04-01), pages 46-56, XP004445091 ISSN: 0021-9517**
• **A.CORMA ET AL.: "AlITQ-6 and TiITQ-6: Synthesis, Characterization, and Catalytic Activity" ANGEW.CHEM., vol. 112, no. 8, 2000, pages 1559-1561, XP002397888 cité dans la demande**

**Description**

La présente invention concerne l'isomérisation de mélanges de composés aromatiques à 8 atomes de carbone, encore appelés « coupes C8 aromatiques ».

Les coupes C8 aromatiques considérées peuvent être définies comme étant choisies parmi :

- les coupes qui comprennent au moins un xylène ;
- les coupes qui comprennent de l'éthylbenzène ; et
- les coupes qui comprennent des mélanges d'au moins un xylène et de l'éthylbenzène.

La présente invention concerne plus particulièrement un procédé d'isomérisation d'une coupe C8 aromatiques visant à maximiser la production de para-xylène.

La catalyse de l'isomérisation de l'éthylbenzène en xylènes nécessite la présence d'un métal du groupe VIII. Les formulations optimisées à base de mordénite et d'un métal du groupe VIII conduisent à des catalyseurs avec lesquels les réactions parasites restent non négligeables. Par exemple, on peut citer l'ouverture de cycles naphténiques, suivie ou non de craquage, ou encore les réactions de dismutation et de transalkylation des aromatiques en C8, qui conduisent à la formation d'aromatiques non recherchés. Il est donc particulièrement intéressant de trouver de nouveaux catalyseurs plus sélectifs.

Parmi les zéolithes utilisées en isomérisation des coupes C8 aromatiques, on trouve la ZSM-5, utilisée seule ou en mélange avec d'autres zéolithes, comme par exemple la mordénite. Ces catalyseurs sont notamment décrits dans les brevets US-A-4 467 129, US-A-4 482 773 et EP-B-13 617. D'autres catalyseurs principalement à base de mordénite ont été décrits par exemple dans les brevets US-A-4 723 051, US-A-4 665 258 et FR-A-2 477 903. Plus récemment, il a été proposé un catalyseur à base d'une zéolithe de type structural EUO (EP-A1-923 987).

Le manque de sélectivité de la mordénite peut être atténué par une optimisation des formulations et/ou par des traitements spécifiques, comme cela a par exemple été décrit dans le brevet FR-B-2 691 914 au nom de la demanderesse. Ces techniques permettent de diminuer les réactions parasites de dismutation.

La demande de brevet WO-A-2005/065 380 décrit l'utilisation d'une zéolithe de type structural MTW en isomérisation des xylènes et de l'éthylbenzène.

Il a été découvert par la demanderesse que, de façon surprenante, un catalyseur, de préférence sous forme d'extrudés ou de billes, utilisé dans les réactions d'isomérisation de coupes C8 aromatiques, contenant au moins une zéolithe ITQ-6, un liant et au moins un métal du groupe VIII de la classification périodique des éléments (avantageusement bien dispersé à la surface du catalyseur et bien réparti macroscopiquement au travers du grain de catalyseur) pouvait conduire à des performances remarquables en isomérisation des xylènes, en termes d'activité, de sélectivité, et également en termes de stabilité temporelle.

Ainsi, l'invention fournit un procédé amélioré d'isomérisation des xylènes dans une coupe C8 aromatique en présence d'un catalyseur qui comprend :

- au moins une zéolithe ITQ-6 ;
- au moins un métal du groupe VIII de la classification périodique des éléments (Handbook of Physics and Chemistry, 76$^{ème}$ édition) ;
- au moins un liant ;
- éventuellement au moins un métal choisi dans le groupe formé par les éléments des groupes IIIA, IVA et VIIB de la classification périodique des éléments ; et
- éventuellement du soufre.

Plus particulièrement, le catalyseur utilisé dans le procédé d'isomérisation des coupes C8 aromatiques selon la présente invention est sous forme de billes ou d'extrudés.

La zéolithe ITQ-6 ne présente à ce jour pas de type structural défini. Il s'agit d'une zéolithe délaminée à partir de la préferriérite lamellaire. Le mode de synthèse de la zéolithe ITQ-6 et ses caractéristiques physico-chimiques ont été décrits notamment dans le brevet EP-B1-1 102 630. Par ailleurs, Corma et al., découvreurs de ce solide, ont enseigné dans un article de la revue Angewandte Chemie Int. Ed. 39 (2000) n°8, p 1499, que la zéolithe ITQ-6 est stable et présente une surface externe importante. En outre, la zéolithe ITQ-6 présente des propriétés catalytiques intéressantes pour les réactions de craquage d'hydrocarbures et notamment de molécules encombrées. Le brevet EP-B1-1 102 630 décrit l'utilisation de la zéolithe ITQ-6 dans des procédés d'isodéparaffinage, d'isomérisation d'alcènes et pour le craquage d'hydrocarbures à température et pression élevée.

La zéolithe entrant dans la composition du catalyseur utilisé dans l'invention est une zéolithe ITQ-6, qui comprend du silicium et au moins un élément T choisi dans le groupe formé par l'aluminium, le fer, le gallium et le bore, de préférence l'aluminium et le bore, dont le rapport atomique Si/T est de 2:1 à 100:1 de préférence de 5:1 à 80:1 et de préférence encore de 5:1 à 50:1.

Dans le catalyseur utilisé dans l'invention, la zéolithe ITQ-6 est au moins en partie, de préférence pratiquement totalement, sous forme acide, c'est-à-dire sous forme hydrogène (H$^+$), la teneur en sodium étant de préférence telle que le rapport atomique Na/T est inférieur à 0,5:1, de préférence inférieur à 0,1:1, de manière encore plus préférée inférieur à 0,02:1.

La zéolithe ITQ-6 entrant dans la composition du catalyseur utilisé dans l'invention présente généralement une surface totale, mesurée par chimisorption d'azote selon la méthode BET, supérieure à 300 m$^2$/g . De préférence, sa surface totale est supérieure à 400 m$^2$/g

Le liant (ou matrice) entrant dans la composition du catalyseur utilisé dans l'invention consiste généralement en au moins un élément choisi dans le groupe formé par les argiles, la magnésie, les alumines, les silices, l'oxyde de titane, l'oxyde de bore, la zircone, les phosphates d'aluminium, les phosphates de titane, les phosphates de zirconium et les silices-alumines. On peut aussi utiliser du charbon. De préférence, le liant est une alumine.

Le métal du groupe VIII présent dans le catalyseur utilisé selon l'invention peut être tout métal choisi parmi le fer, le cobalt, le nickel, le ruthénium, le rhodium, le palladium, l'osmium, l'iridium et le platine. Il consiste de préférence en un métal noble tel que le palladium ou le platine. De manière encore plus préférée, c'est le platine.

Selon la méthode mise en oeuvre pour le dépôt, comme il sera indiqué ci-après, le métal noble du groupe VIII peut être déposé de manière prépondérante sur la zéolithe ou sur le liant.

Avantageusement, ce métal sera bien dispersé sur la surface du catalyseur et bien réparti macroscopiquement au travers du grain de catalyseur.

Le catalyseur utilisé selon l'invention peut encore contenir, à titre facultatif, au moins un métal choisi dans le groupe formé par les éléments des groupes IIIA, IVA et VIIB de la classification périodique des éléments et choisi par exemple parmi le gallium, l'indium, l'étain et le rhénium. Ce métal est de préférence l'indium, l'étain ou le rhénium.

Dans la composition du catalyseur utilisé dans l'invention, la zéolithe ITQ-6 entre plus particulièrement à raison de 1 à 90 %, de préférence de 3 à 80 % et, de manière encore plus préférée, de 4 à 60 % en poids par rapport au poids de catalyseur. Le métal du groupe VIII, déposé sur la zéolithe ou sur le liant, représente de 0,01 à 4 %, de préférence de 0,05 à 2,0 %, en poids par rapport au poids de catalyseur. Le liant constitue le complément à 100 %.

Lorsque le catalyseur utilisé dans l'invention contient au moins un métal choisi parmi les métaux des groupes IIIA, IVA et VIIB, la teneur en celui-ci peut aller jusqu'à 2 % en poids par rapport au poids de catalyseur. Elle est alors avantageusement de 0,01 à 2 %, de préférence de 0,05 à 1,0 % en poids.

Lorsque le catalyseur utilisé dans l'invention contient du soufre, la teneur en celui-ci peut être telle que le rapport du nombre d'atomes de soufre sur le nombre d'atomes de métal du groupe VIII déposés aille jusqu'à 2:1. Il est alors avantageusement de 0,5:1 à 2:1.

La préparation du catalyseur utilisé dans l'invention peut être effectuée par toute méthode connue de l'homme du métier.

Le métal du groupe VIII peut être introduit sur la zéolithe ou sur le liant avant ou après mise en forme. Lorsqu'on introduit plusieurs métaux, ceux-ci peuvent être introduits, soit tous de la même façon, soit par des techniques différentes, à tout moment de la préparation, avant ou après mise en forme et dans n'importe quel ordre.

Cependant, la méthode préférée de préparation du catalyseur utilisé dans l'invention consiste à mettre en forme le mélange de la zéolithe et du liant avant le dépôt du métal du groupe VIII et éventuellement celui du métal des groupes IIIA, IVA et VIIB.

La mise en forme consiste plus particulièrement à malaxer la zéolithe ITQ-6 dans un gel humide de matrice (obtenu généralement par mélange d'au moins un acide et d'une poudre de matrice), par exemple l'alumine, pendant une durée nécessaire à l'obtention d'une bonne homogénéité de la pâte, soit par exemple pendant une dizaine de minutes, puis à passer la pâte ainsi obtenue à travers une filière pour former des extrudés, par exemple de diamètre de 0,4 à 4 mm.

La mise en forme est généralement suivie d'un séchage (par exemple pendant quelques heures à environ 120°C en étuve) et d'une calcination, généralement à une température de 250°C à 600°C (par exemple pendant deux heures à environ 400°C).

Après calcination, au moins un élément du groupe VIII est introduit de préférence par dépôt sélectif sur le mélange zéolithe-liant par tout procédé connu de l'homme du métier. Un tel dépôt est par exemple effectué par la technique d'imprégnation à sec, la technique d'imprégnation par excès, ou plus particulièrement par échange ionique.

Le dépôt est avantageusement opéré de façon telle que la dispersion du(des)dit(s) élément(s), déterminée par chimisorption, soit de 50 % à 100 %, de préférence de 60 % à 100 % et, de manière encore plus préférée, de 70 % à 100 %.

Il est également préféré d'obtenir une bonne répartition du(des)dit(s) élément(s) dans le catalyseur mis en forme. Cette répartition est caractérisée par son profil obtenu par microsonde de Castaing. Le rapport des concentrations de chaque élément du groupe VIII au coeur du grain par rapport au bord de ce même grain, défini comme étant le coefficient de répartition, est avantageusement de 0,7:1 à 1,3:1, de préférence de 0,8:1 à 1,2:1.

Tous les précurseurs conviennent pour le dépôt de ces éléments. A titre indicatif, le platine peut être introduit sous forme d'acide hexachloroplatinique, mais, pour tout métal noble du groupe VIII, on peut également utiliser des composés ammoniaqués ou des composés tels que par exemple le chloroplatinate d'ammonium, le dichlorure de platine dicarbonyle, l'acide hexahydroxyplatinique, le chlorure de palladium ou le nitrate de palladium.

Comme cela sera indiqué plus loin, le choix du précurseur t du mode de dépôt se fait en fonction de la localisation voulue du métal dans (ou sur) la zéolithe et/ou dans (ou sur) le liant.

Le contrôle de certains paramètres mis en oeuvre lors du dépôt, en particulier la nature du précurseur du (des) élément(s) du groupe VIII utilisé(s) permet d'orienter le dépôt dudit (desdits) élément(s) majoritairement sur le liant ou sur la zéolithe.

Ainsi, pour introduire le métal du groupe VIII, par exemple le platine ou le palladium, on peut mettre en oeuvre un échange anionique avec de l'acide hexachloroplatinique et/ou de l'acide hexachloropalladique, en présence d'un agent compétiteur, par exemple l'acide chlorhydrique, le dépôt étant en général suivi d'une calcination, par exemple pendant environ 2 heures à environ 400°C.

Avec de tels précurseurs, le métal est déposé majoritairement sur le liant et il présente une bonne dispersion et une bonne répartition macroscopique à travers le grain de catalyseur.

On peut aussi envisager de déposer le métal du groupe VIII par échange cationique. Ainsi, dans le cas du platine, le précurseur peut être par exemple choisi parmi :

- les composés ammoniaqués tels que les sels de platine (II) tétrammines de formule Pt(NH3)4X2, les sels de platine (IV) hexammines de formule $Pt(NH_3)_6X_4$; les sels de platine (IV) halogénopentammines de formule $(PtX(NH_3)_5)X_3$ ; les sels de platine N-tétrahalogénodiammines de formule $PtX_4(NH_3)_2$ ; et
- les composés halogénés de formule $H(Pt(acac)_2X)$ ;

X étant un halogène choisi dans le groupe formé par le chlore, le fluor, le brome et l'iode, X étant de préférence le chlore, et "acac" représentant le groupe acétylacétonate (de formule brute $C_5H_7O_2$), dérivé de l'acétylacétone.

Dans ce cas, le métal du groupe VIII sera déposé majoritairement sur la zéolithe. Il présente une bonne dispersion et une bonne répartition macroscopique à travers le grain de catalyseur.

Lorsque l'on souhaite déposer sur le catalyseur plusieurs éléments du groupe VIII, ces métaux peuvent être introduits, soit tous de la même façon, soit par des techniques différentes, et dans n'importe quel ordre.

L'introduction du métal noble de la famille du platine est de préférence effectuée par imprégnation à l'aide d'une solution aqueuse ou organique de l'un des précurseurs cités ci-dessus.

Parmi les solvants organiques utilisables, on peut citer les hydrocarbures paraffiniques, naphténiques ou aromatiques contenant par exemple de 6 à 12 atomes de carbone par molécule, et les composés organiques halogénés contenant par exemple de 1 à 12 atomes de carbone par molécule. On peut citer par exemple le n-heptane, le méthylcyclohexane, le toluène et le chloroforme. On peut aussi utiliser les mélanges de solvants.

Dans le catalyseur utilisé dans l'invention, lorsqu'on envisage d'introduire au moins un métal des groupes IIIA, IVA et VIIB, toutes les techniques de dépôt connues de l'homme du métier et tous les précurseurs peuvent convenir.

On peut ajouter les éléments du groupe VIII et ceux des groupes IIIA, IVA et VIIB, soit séparément à n'importe quelle étape de la préparation dudit catalyseur, soit simultanément dans au moins une étape unitaire. Lorsqu'au moins un élément des groupes IIIA, IVA et VIIB est ajouté séparément, il est préférable qu'il soit ajouté après l'élément du groupe VIII.

Le métal additionnel peut être introduit par l'intermédiaire de composés tels que par exemple les chlorures, les bromures et les nitrates d'éléments des groupes IIIA, IVA et VIIB. Par exemple dans le cas de l'indium, on utilisera avantageusement le nitrate ou le chlorure et dans le cas du rhénium, l'acide perrhénique.

Ce métal peut également être introduit sous la forme d'au moins un composé organique choisi dans le groupe constitué par les complexes dudit métal, en particulier les complexes polycétoniques du métal et les hydrocarbylmétaux tels que les alkyles, les cycloalkyles, les aryles, les alkylaryles et les arylalkyles métaux. Dans ce dernier cas, l'introduction du métal est avantageusement effectuée à l'aide d'une solution du composé organométallique dudit métal dans un solvant organique. On peut également employer des composés organohalogénés du métal. Comme composés organiques de métaux, on peut citer en particulier le tétrabutylétain, dans le cas de l'étain, et le triphénylindium, dans le cas de l'indium.

Si le métal additionnel est introduit avant le métal du groupe VIII, le composé du métal utilisé est généralement choisi dans le groupe constitué par l'halogénure, le nitrate, l'acétate, le tartrate, le carbonate et l'oxalate du métal. L'introduction est alors avantageusement effectuée en solution aqueuse. Mais il peut également être introduit à l'aide d'une solution d'un composé organométallique du métal par exemple le tétrabutylétain. Dans ce cas, avant de procéder à l'introduction d'au moins un métal du groupe VIII, on procédera à une calcination sous air.

De plus, des traitements intermédiaires tels que par exemple une calcination et/ou une réduction peuvent être appliqués entre les dépôts successifs des différents métaux.

La préparation du catalyseur se termine généralement par une calcination, habituellement à une température d'environ 250°C à 600°C, pour une durée d'environ 0,5 à 10 heures, de préférence précédée d'un séchage, par exemple à l'étuve, à une température allant de la température ambiante à 250°C, de préférence de 40°C à 200°C. Ladite étape de séchage est de préférence menée pendant la montée en température nécessaire pour effectuer ladite calcination.

On peut mettre en oeuvre une réduction préalable du catalyseur *ex situ,* sous courant d'hydrogène, par exemple à une température de 450°C à 600°C, pendant une durée de 0,5 à 4 heures.

Dans le cas où le catalyseur ne contient pas de soufre, une réduction du métal sous hydrogène est réalisée *in situ* avant injection de la charge.

Dans le cas où le catalyseur utilisé dans l'invention contient du soufre, le soufre est de préférence introduit sur le catalyseur mis en forme, calciné, contenant le ou les métaux cités précédemment, soit *in situ* avant la réaction catalytique, soit *ex situ.* La sulfuration éventuelle intervient après la réduction. Dans le cas d'une sulfuration *in situ,* la réduction, si le catalyseur n'a pas été préalablement réduit, intervient avant la sulfuration. Dans le cas d'une sulfuration *ex situ,* on effectue la réduction puis la sulfuration. La sulfuration s'effectue en présence d'hydrogène en utilisant tout agent sulfurant bien connu de l'homme de métier, tel que par exemple le sulfure de diméthyle ou le sulfure d'hydrogène. Par exemple, le catalyseur est traité avec une charge contenant du sulfure de diméthyle en présence d'hydrogène, avec une concentration telle que le rapport atomique soufre/métal soit de 1,5. Le catalyseur est ensuite maintenu pendant environ 3 heures à environ 400°C sous débit d'hydrogène avant l'injection de la charge.

Le catalyseur utilisé dans l'invention peut être mis en oeuvre dans les réactions d'isomérisation d'une coupe C8 aromatique, comprenant par exemple soit uniquement un mélange de xylènes, soit uniquement de l'éthylbenzène, soit un mélange de xylène(s) et d'éthylbenzène. Ledit procédé est mis en oeuvre généralement selon les conditions opératoires suivantes :

- une température de 300°C à 500°C de préférence de 320°C à 450°C et de manière encore plus préférée de 340°C à 430°C ;
- une pression partielle d'hydrogène de 0,3 à 1,5 MPa de préférence de 0,4 et 1,2 MPa et de manière encore préférée de 0,7 à 1,2 MPa ;
- une pression totale de 0,45 à 1,9 MPa de préférence de 0,6 à 1,5 MPa ; et
- une vitesse spatiale d'alimentation, exprimée en kilogramme de charge introduite par kilogramme de catalyseur et par heure, de 0,25 à 30 $h^{-1}$ de préférence de 1 à 10 $h^{-1}$ et de manière encore préférée de 2 à 6 $h^{-1}$.

Le catalyseur utilisé dans l'invention, mis sous forme de billes ou d'extrudés mécaniquement résistants, constitué d'au moins une zéolithe ITQ-6, d'au moins un liant, d'au moins un métal choisi parmi les éléments du groupe VIII de la classification périodique des éléments, ledit métal étant bien dispersé sur la surface du catalyseur et bien réparti macroscopiquement à travers le grain de catalyseur présente d'excellentes performances catalytiques en transformations d'hydrocarbures, en termes d'activité, de sélectivité et de stabilité temporelle, comme par exemple en isomérisation des coupes C8 aromatiques, c'est-à-dire de mélanges constitués de xylènes et éventuellement d'éthylbenzène.

Les exemples suivants illustrent l'invention sans toutefois en limiter la portée.

**Exemple 1 :** Préparation du catalyseur contenant de la zéolithe ITQ-6 et 0,3 % poids de platine.

La zéolithe de départ est une ITQ-6 de rapport Si/Al = 15, sous forme H$^+$. Cette zéolithe est ensuite mise en forme par extrusion (diamètre d'extrusion = 1,4 mm) avec un gel d'alumine de manière à obtenir, après séchage et calcination sous air sec, un support S1 qui contient 20 % poids de zéolithe ITQ-6 sous forme hydrogène et 80 % poids d'alumine.

Ce support S1 est soumis à un échange anionique avec de l'acide hexachloroplatinique en présence d'un agent compétiteur (acide chlorhydrique), de manière à déposer 0,3 % poids de platine par rapport au catalyseur. Le solide humide est ensuite séché à 120°C pendant 12 heures et calciné sous un débit d'air sec à la température de 500°C pendant une heure.

Le catalyseur A ainsi obtenu contient, en poids, 20 % de ITQ-6- sous forme hydrogène, 79,7 % d'alumine et 0,3 % de platine.

**Exemple 2 :** Évaluation des propriétés catalytiques du catalyseur A en isomérisation d'une coupe C8 aromatique.

La coupe C8 aromatique à isomériser contient principalement du méta-xylène, de l'ortho-xylène et de l'éthylbenzène.

Les conditions opératoires de l'isomérisation sont les suivantes :

- température : 390°C ;
- pression totale : 15 bar (1 bar = 0,1 MPa) ;

- pression partielle d'hydrogène : 12 bar.

Le catalyseur A obtenu comme décrit dans l'Exemple 1 est réduit à 450°C pendant 2 heures sous débit d'hydrogène.

Le catalyseur est préalablement traité avec une charge contenant du disulfure de diméthyle (DMDS) en présence d'hydrogène, avec une concentration telle que le rapport atomique soufre/métal soit de 1,5. Le catalyseur est ensuite maintenu pendant 3 heures à 400°C sous débit d'hydrogène, puis la charge est injectée.

Le catalyseur a été évalué en terme d'activité (par les approches à l'équilibre du para-xylène et de l'éthylbenzène et par la conversion de l'éthylbenzène), et en terme de sélectivité (par les pertes nettes à iso-approche à l'équilibre du para-xylène).

De plus, les réactions parasites conduisent à trois types de pertes : les pertes vers les paraffines résultant essentiellement de réactions d'ouverture de cycles naphténiques suivies de craquage, les pertes vers les aromatiques formés par réactions de dismutation et de transalkylation des aromatiques à 8 atomes de carbone (AC8), et enfin les pertes vers les naphtènes dont les naphtènes à 8 atomes de carbone (N8) dus à l'hydrogénation des aromatiques. Les N8 pouvant être recyclés, on comparera les pertes par craquage et dismutation/transalkylation incluant les naphtènes autres que N8 (dont la somme constitue les pertes nettes) en prenant une base 100, pour chacune de ces pertes, pour le catalyseur A non conforme à l'invention.

Pour le calcul des approches à l'équilibre (AEQ), les concentrations en éthylbenzène (% EB) sont exprimées par rapport aux quatre isomères AC8, et celles en para-xylène (% pX) par rapport aux trois isomères xylènes.

Les approches à l'équilibre (AEQ) sont définies de la manière suivante :

$$\text{pX AEQ (\%)} = 100 \times (\% \text{ pX}_{effluent} - \% \text{ pX}_{charge}) / (\% \text{ pX}_{équilibre} - \% \text{ pX}_{charge})$$

$$\text{EB AEQ (\%)} = 100 \times (\% \text{ EB}_{effluent} - \% \text{ EB}_{charge}) / (\% \text{ EB}_{équilibre} - \% \text{ EB}_{charge})$$

Les pertes par craquage (P1 en % poids) sont des pertes en AC8 sous forme de paraffines (PAR) de C1 à C8 :

$$\frac{100 \times [(\% \text{ PAR}_{effluent} \times \text{poids d'effluent}) - (\% \text{ PAR}_{charge} \times \text{poids de charge})]}{(\% \text{ AC8}_{charge} \times \text{poids de charge})}$$

Les pertes par dismutation/transalkylation (P2 en % poids) sont des pertes en AC8 sous forme de naphtènes autres que N8, de toluène, de benzène et de C9+ aromatiques (OAN) :

$$\frac{100 \times [(\% \text{ OAN}_{effluent} \times \text{poids d'effluent}) - (\% \text{ OAN}_{charge} \times \text{poids de charge})]}{(\% \text{ AC8}_{charge} \times \text{poids de charge})}$$

La somme des pertes P1 et P2 représente les pertes nettes.

On a obtenu les résultats suivants :

- pX AEQ (%) : 98,2
- EB AEQ (%) : 91,3
- Conversion EB (%) : 56,4
- Pertes nettes (% poids) : 5,4

Ces résultats montrent que le catalyseur A présente, selon l'invention, de bonnes performances dans l'isomérisation d'une coupe C8 aromatique.

**Revendications**

1. Procédé d'isomérisation d'une coupe C8 aromatique en présence d'un catalyseur, dans lequel ledit catalyseur comprend :

   - au moins une zéolithe ITQ-6 ;
   - au moins un métal du groupe VIII de la classification périodique des éléments ;
   - au moins un liant ;
   - éventuellement au moins un métal choisi dans le groupe formé par les éléments des groupes IIIA, IVA et VIIB de la classification périodique des éléments ; et
   - éventuellement du soufre.

2. Procédé selon la revendication 1 dans lequel la zéolithe ITQ-6 comprend du silicium et au moins un élément T choisi dans le groupe formé par l'aluminium, le fer, le gallium et le bore, dont le rapport atomique Si/T est de 2:1 à 100:1, ladite zéolithe étant au moins en partie sous forme acide.

3. Procédé selon la revendication 2 dans lequel la zéolithe ITQ-6 présente une teneur en sodium correspondant à un rapport atomique Na/T inférieur à 0,5:1.

4. Procédé selon l'une des revendications 1 à 3 dans lequel la zéolithe ITQ-6 présente une surface totale, mesurée par chimisorption d'azote selon la méthode BET, supérieure à 300 $m^2$/g.

5. Procédé selon l'une des revendications 1 à 4 dans lequel le métal du groupe VIII est choisi dans le groupe formé par le platine et le palladium.

6. Procédé selon la revendication 5 dans lequel le métal du groupe VIII est le platine.

7. Procédé selon l'une des revendications 1 à 6 dans lequel le métal des groupes IIIA, IVA et VIIB est le gallium, l'indium, l'étain ou le rhénium.

8. Procédé selon l'une des revendications 1 à 7 dans lequel :

   - la zéolithe ITQ-6 représente de 1 à 90 % en poids du catalyseur ;
   - le métal du groupe VIII représente de 0,01 à 4 % en poids du catalyseur ;
   - le complément à 100 % en poids du catalyseur étant constitué par au moins un liant.

9. Procédé selon l'une des revendications 1 à 8 dans lequel le coefficient de répartition du métal du groupe VIII, déterminé sur un grain de catalyseur, correspond à un rapport de la concentration du métal du groupe VIII au coeur du grain par rapport au bord du même grain de 0,7:1 à 1,3:1.

10. Procédé selon l'une des revendications 1 à 9 dans lequel la dispersion du métal du groupe VIII sur le mélange zéolithe-liant, déterminée par chimisorption, est de 50 % à 100%.

11. Procédé selon l'une des revendications 1 à 10 dans lequel le métal du groupe VIII est déposé principalement sur le liant.

12. Procédé selon l'une des revendications 1 à 10 dans lequel le métal du groupe VIII est déposé principalement sur la zéolithe.

13. Procédé selon l'une des revendications 1 à 12 dans lequel le métal des groupes IIIA, IVA et VIIB est présent à une teneur allant jusqu'à 2 % en poids par rapport au poids du catalyseur.

14. Procédé selon l'une des revendications 1 à 13 dans lequel ledit catalyseur comprend en outre du soufre à une teneur correspondant à un rapport du nombre d'atomes de soufre sur le nombre d'atomes de métal du groupe VIII allant jusqu'à 2:1.

15. Procédé selon l'une des revendications 1 à 14 dans lequel la réaction d'isomérisation est mise en oeuvre dans les conditions opératoires suivantes :

- une température de 300°C à 500°C ;
- une pression partielle d'hydrogène de 0,3 à 1,5 MPa ;
- une pression totale de 0,45 à 1,9 MPa ; et
- une vitesse spatiale d'alimentation, exprimée en kilogramme de charge introduite par kilogramme de catalyseur et par heure, de 0,25 à 30 h$^{-1}$.

16. Procédé l'une des revendications 1 à 15 dans lequel ladite coupe C8 aromatique est choisie parmi :

- les coupes qui comprennent au moins un xylène ;
- les coupes qui comprennent de l'éthylbenzène ; et
- les coupes qui comprennent des mélanges d'au moins un xylène et de l'éthylbenzène.

**Claims**

1. A process for isomerizing an aromatic C8 cut in the presence of a catalyst, in which said catalyst comprises:

   • at least one ITQ-6 zeolite;
   • at least one metal from group VIII of the periodic table;
   • at least one binder;
   • optionally, at least one metal selected from the group formed by elements from groups IIIA, IVA and VIIB of the periodic table; and
   • optionally, sulphur.

2. A process according to claim 1, in which the ITQ-6 zeolite comprises silicon and at least one element T selected from the group formed by aluminium, iron, gallium and boron, wherein the Si/T atomic ratio is 2:1 to 100:1, said zeolite being at least partially in the acid form.

3. A process according to claim 2, in which the ITQ-6 zeolite has a sodium content corresponding to a Na/T atomic ratio of less than 0.5:1.

4. A process according to one of claims 1 to 3, in which the ITQ-6 zeolite has a total surface area, measured by nitrogen chemisorption using the BET method, of more than 300 m$^2$/g.

5. A process according to one of claims 1 to 4, in which the group VIII metal is selected from the group formed by platinum and palladium.

6. A process according to claim 5, in which the group VIII metal is platinum.

7. A process according to one of claims 1 to 6, in which the metal from groups IIIA, IVA and VIIB is gallium, indium, tin or rhenium.

8. A process according to one of claims 1 to 7, in which:

   • the ITQ-6 zeolite represents 1% to 90% by weight of the catalyst;
   • the group VIII metal represents 0.01% to 4% by weight of catalyst;
   • the complement to 100% by weight of the catalyst being constituted by at least one binder.

9. A process according to one of claims 1 to 8, in which the distribution coefficient of the group VIII metal, determined for a grain of catalyst, corresponds to a ratio of the concentration of the group VIII metal in the core of the grain with respect to the edge of that grain of 0.7:1 to 1.3:1.

10. A process according to one of claims 1 to 9, in which the dispersion of the group VIII metal on the zeolite-binder mixture, determined by chemisorption, is 50% to 100%.

11. A process according to one of claims 1 to 10, in which the group VIII metal is principally deposited on the binder.

12. A process according to one of claims 1 to 10, in which the group VIII metal is principally deposited on the zeolite.

13. A process according to one of claims 1 to 12, in which the metal from groups IIIA, IVA and VIIB is present in an amount of up to 2% by weight with respect to the weight of catalyst.

14. A process according to one of claims 1 to 13, in which said catalyst further comprises sulphur in an amount corresponding to a ratio of the number of sulphur atoms to the number of atoms of group VIII metal of up to 2:1.

15. A process according to one of claims 1 to 14, in which the isomerization reaction is carried out under the following operating conditions:

- a temperature of 300°C to 500°C;
- a partial pressure of hydrogen of 0.3 to 1.5 MPa;
- a total pressure of 0.45 to 1.9 MPa; and
- an hourly space velocity, expressed in kilograms of feed introduced per kilogram of catalyst per hour, of 0.25 to 30 $h^{-1}$.

16. A process according to one of claims 1 to 15, in which said aromatic C8 cut is selected from:

- cuts which comprise at least one xylene;
- cuts which comprise ethylbenzene; and
- cuts which comprise mixtures of at least one xylene and ethylbenzene.

**Patentansprüche**

1. Verfahren zur Isomerisierung eines aromatischen C8-Schnitts in Gegenwart eines Katalysators, wobei der Katalysator Folgendes umfasst:

- mindestens einen Zeolithen ITQ-6;
- mindestens ein Metall der Gruppe VIII des Periodensystems der Elemente;
- mindestens ein Bindemittel;
- gegebenenfalls mindestens ein Metall ausgewählt aus der Gruppe gebildet aus den Elementen der Gruppen IIIA, IVA und VIIB des Periodensystems der Elemente; und
- gegebenenfalls Schwefel.

2. Verfahren nach Anspruch 1, wobei der Zeolith ITQ-6 Silicium und mindestens ein Element T umfasst, ausgewählt aus der Gruppe gebildet aus Aluminium, Eisen, Gallium und Bor, wobei das Si/T-Atomverhältnis 2:1 bis 100:1 beträgt, wobei der Zeolith mindestens teilweise Säureform aufweist.

3. Verfahren nach Anspruch 2, wobei der Zeolith ITQ-6 einen Natriumgehalt aufweist, der einem Na/T-Atomverhältnis von kleiner als 0,5:1 entspricht.

4. Verfahren nach einem der Ansprüche 1 bis 3, wobei der Zeolith ITQ-6 eine Gesamtoberfläche, gemessen mittels Stickstoffchemisorption gemäß der BET-Methode, von mehr als 300 $m^2$/g aufweist.

5. Verfahren nach einem der Ansprüche 1 bis 4, wobei das Metall der Gruppe VIII ausgewählt ist aus der Gruppe gebildet aus Platin und Palladium.

6. Verfahren nach Anspruch 5, wobei das Metall der Gruppe VIII Platin ist.

7. Verfahren nach einem der Ansprüche 1 bis 6, wobei das Metall der Gruppen IIIA, IVA und VIIB Gallium, Indium, Zinn oder Rhenium ist.

8. Verfahren nach einem der Ansprüche 1 bis 7, wobei:

- der Zeolith ITQ-6 1 bis 90 Gew.-% des Katalysators darstellt;
- das Metall der Gruppe VIII 0,01 bis 4 Gew.-% des Katalysators darstellt;
- wobei die Ergänzung auf 100 Gew.-% des Katalysators aus mindestens einem Bindemittel besteht.

**9.** Verfahren nach einem der Ansprüche 1 bis 8, wobei der Verteilungskoeffizient des Metalls der Gruppe VIII, der an einem Katalysatorkorn bestimmt wird, einem Verhältnis der Konzentration des Metalls der Gruppe VIII im Kern des Korns bezogen auf den Rand des gleichen Korns von 0,7:1 bis 1,3:1 entspricht.

**10.** Verfahren nach einem der Ansprüche 1 bis 9, wobei die Dispersion des Metalls der Gruppe VIII auf dem Zeolith-Bindemittel-Gemisch, bestimmt mittels Chemisorption, 50 % bis 100 % beträgt.

**11.** Verfahren nach einem der Ansprüche 1 bis 10, wobei das Metall der Gruppe VIII im Wesentlichen auf dem Bindemittel abgeschieden wird.

**12.** Verfahren nach einem der Ansprüche 1 bis 10, wobei das Metall der Gruppe VIII im Wesentlichen auf dem Zeolithen abgeschieden wird.

**13.** Verfahren nach einem der Ansprüche 1 bis 12, wobei das Metall der Gruppen IIIA, IVA und VIIB in einem Anteil von bis zu 2 Gew.-% vorhanden ist, bezogen auf das Gewicht des Katalysators.

**14.** Verfahren nach einem der Ansprüche 1 bis 13, wobei der Katalysator ferner Schwefel in einem Anteil umfasst, der einem Verhältnis der Anzahl der Schwefelatome zur Anzahl der Gruppe-VIII-Metallatome von bis zu 2:1 entspricht.

**15.** Verfahren nach einem der Ansprüche 1 bis 14, wobei die Isomerisierungsreaktion unter den folgenden Betriebsbedingungen durchgeführt wird:

- einer Temperatur von 300°C bis 500 °C;
- einem Wasserstoffpartialdruck von 0,3 bis 1,5 MPa;
- einem Gesamtdruck von 0,45 bis 1,9 MPa; und
- einer Versorgungsraumgeschwindigkeit, ausgedrückt in Kilogramm eingeführter Beschickung pro Kilogramm Katalysator und pro Stunde von 0,25 bis 30 $h^{-1}$.

**16.** Verfahren nach einem der Ansprüche 1 bis 15, wobei der aromatische C8-Schnitt ausgewählt ist aus:

- Schnitten, die mindestens ein Xylol umfassen;
- Schnitten, die Ethylbenzol umfassen; und
- Schnitten, die Gemische aus mindestens einem Xylol und Ethylbenzol umfassen.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 4467129 A
- US 4482773 A
- EP 13617 B
- US 4723051 A
- US 4665258 A
- FR 2477903 A
- EP 923987 A1
- FR 2691914 B
- WO 2005065380 A
- EP 1102630 B1

**Littérature non-brevet citée dans la description**

- *Angewandte Chemie Int. Ed.,* 2000, vol. 39 (8), 1499